(19) Europäisches Patentamt
European Patent Office
Office européen des brevets

(11) Veröffentlichungsnummer: **0 333 024 B1**

(12) # EUROPÄISCHE PATENTSCHRIFT

(45) Veröffentlichungstag der Patentschrift: **19.10.94**

(51) Int. Cl.$^5$: **C07D 313/00**, C12N 1/14, A61K 31/335

(21) Anmeldenummer: **89104160.0**

(22) Anmeldetag: **09.03.89**

(54) **Neue 10-Ring-Lactone, Verfahren zu ihrer Herstellung und ihre Verwendung.**

(30) Priorität: **15.03.88 DE 3808492**

(43) Veröffentlichungstag der Anmeldung:
**20.09.89 Patentblatt 89/38**

(45) Bekanntmachung des Hinweises auf die Patenterteilung:
**19.10.94 Patentblatt 94/42**

(84) Benannte Vertragsstaaten:
**BE CH DE FR GB IT LI NL**

(56) Entgegenhaltungen:

**THE JOURNAL OF ORGANIC CHEMISTRY, Band 44, Nr. 12, 8. Juni 1979, Seiten 2008-2012, American Chemical Society, Washington, D.C., US; T. WAKAMATSU et al.:"Naturally occurring ten-membered lactones: Total synthesis of (+)-diplodialideA,(+)-diplodialide C, and (+)-decan-9-olide"**

**H. Dörfelt, Lexikon der Mykologie, G. Fischer Verlag, 1989, Seite 95 und 205**

(73) Patentinhaber: **HOECHST AKTIENGESELLSCHAFT**

**D-65926 Frankfurt (DE)**

(72) Erfinder: **Wink, Joachim, Dr.**
**Bieberer Strasse 133**
**D-6050 Offenbach (DE)**
Erfinder: **Grabley, Susanne, Dr.**
**Hölderlinstrasse 7**
**D-6240 Königstein/Taunus (DE)**
Erfinder: **Seibert, Gerhard, Prof. Dr.**
**Gläserweg 21**
**D-6100 Darmstadt (DE)**
Erfinder: **Hütter, Klaus, Dr.**
**Robert-Stolz-Strasse 3**
**D-6232 Bad Soden am Taunus (DE)**
Erfinder: **Zeeck, Axel, Prof.Dr.**
**Brüder-Grimm-Allee 22**
**D-3400 Göttingen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 333 024 B1

## Beschreibung

Antibiotika aus Penicillium sind, wie z.B. das Penicillin, schon lange bekannt.

Aus J. Org. Chem. 44, S. 2008, 1979, ist bekannt, daß der Mikroorganismus Diplodia pinea 10-Ring-Lactone synthetisiert. Diplodia pinea gehört zu den Fungi imperfecti. Die Fungi imperfecti umfassen etwa 1000 Arten, die erhebliche morphologische Unterschiede aufweisen. Auch Penicillium sp. gehört zu den Fungi imperfecti. Aufgrund morphologischer Unterschiede werden die o.g. Mikroorganismen jedoch in unterschiedliche Formmassen eingeordnet (Formklasse Hyphomycetes: Fungi imperfecti; Formklasse Coelomycetes: Diplodia pinea).

Es wurde gefunden, daß auch Penicillium-Stämme 10-Ring-Lactone synthetisieren können. Diese Verbindungen besitzen pharmakologische und damit therapeutische Wirksamkeit und können insbesondere vorteilhaft als antibakterielles Mittel angewendet werden.

Die Erfindung betrifft somit:

1. Eine Verbindung der allgemeinen Formel I,

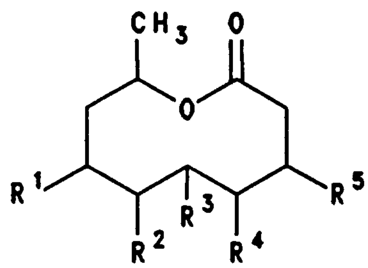

I

in der unabhängig voneinander

| | |
|---|---|
| $R^1$ | Wasserstoff oder Hydroxyl, |
| $R^2$ | Hydroxyl oder |
| $R^1$ und $R^2$ | zusammen mit den tragenden C-Atomen einen Oxiranring darstellen, |
| $R^3$ | Hydroxyl oder |
| $R^2$ und $R^3$ | gemeinsam eine Doppelbindung darstellen, |
| $R^4$ | Wasserstoff oder Hydroxyl oder |
| $R^3$ und $R^4$ | gemeinsam eine Doppelbindung darstellen und |
| $R^5$ | Wasserstoff, Hydroxyl oder eine Oxogruppe bedeuten. |

2. Ein Verfahren zur Herstellung der Verbindung der allgemeinen Formel I nach Anspruch 1, dadurch gekennzeichnet, daS Penicillium sp. DSM 4209 und/oder DSM 4210 in einem Nährmedium kultiviert wird, bis sich die Verbindung der allgemeinen Formel I in der Kultur anhäuft.

3. Eine Verwendung der Verbindung der allgemeinen Formel I nach Anspruch 1 zur Herstellung eines Arzneimittels für die therapeutische Anwendung.

Im folgenden wird die Erfindung detailliert beschrieben, insbesondere in ihren bevorzugten Ausführungsformen. Ferner wird die Erfindung in den Patentansprüchen definiert.

Die Erfindung wird bevorzugt mit Penicillium sp. DSM 4209 und DSM 4210 durchgeführt. Diese Stämme wurden aus einer Erdprobe aus Bryce Canyon, Utah, USA isoliert und bei der Deutschen Sammlung von Mikroorganismen nach den Regeln des Budapester Vertrags am 13.8.1987 unter obengenannter Nummer hinterlegt. Konidien und Sporen des Pilzes wurden wie folgt charakterisiert:

Konidien: Monoverticilliata
Sporenoberfläche: stachelig
Sporenfarbe: graugrün.

In einer Nährlösung, die eine Kohlenstoffquelle und eine Stickstoffquelle sowie die üblichen anorganischen Salze enthält, produziert Penicillium spec., bevorzugt DSM 4209 und DSM 4210, die Verbindung der allgemeinen Formel I. Anstelle der Stämme DSM 4209 oder 4210 können natürlich auch deren Mutanten und Varianten eingesetzt werden, soweit sie diese Verbindung synthetisieren. Solche Mutanten können in an sich bekannter Weise durch physikalische Mittel, beispielsweise Bestrahlung, wie mit Ultraviolet- oder Röntgenstrahlen, oder chemische Mutagene, wie beispielsweise Ethylmethansulfonat (EMS), 2-Hydroxy-4-methoxy-benzophenon (MOB) oder N-Methyl-N'-nitro-N-nitrosoguanidin (MNNG) erzeugt werden.

Als bevorzugte Kohlenstoffquellen für die aerobe Fermentation eignen sich assimilierbare Kohlenhydrate und Zuckeralkohole, wie Glukose, Laktose oder D-Mannit sowie kohlenhydrathaltige Naturprodukte, wie Malzextrakt. Als stickstoffhaltige Nährstoffe kommen in Betracht: Aminosäuren, Peptide und Proteine sowie

deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anderen organischen Salzen kann die Nährlösung beispielsweise Chloride, Carbonate, Sulfate oder Phosphate der Alkali- oder Erdalkalimetalle, Eisen, Zink und Mangan enthalten.

Die Bildung der Verbindung der Formel I verläuft besonders gut in einer Nährlösung, die etwa 0,2 bis 5 %, bevorzugt 1 bis 4 %, Malzextrakt, 0,02 bis 0,5 %, bevorzugt 0,1 bis 0,4 % Hefeextrakt, 0,1 bis 5 %, bevorzugt 0,5 bis 2 % Glucose und 0,005 bis 0,2 %, bevorzugt 0,01 bis 0,1 % Ammoniumsalz enthält, jeweils bezogen auf das Gewicht der gesamten Nährlösung. Die Kultivierung erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 18 bis 35 °C, vorzugsweise bei etwa 25 bis 30 °C, insbesondere bei 27 bis 28 °C durchgeführt werden. Der pH-Bereich sollte zwischen 6 und 8 liegen, vorteilhaft zwischen 6,5 und 7,5. Man kultiviert den Mikroorganismus unter diesen Bedingungen im allgemeinen über einen Zeitraum von 60 bis 170 Stunden, bevorzugt 100 bis 150 Stunden.

Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z. B., indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar, wachsen läßt.

Der Fermentationsverlauf kann anhand des pH-Wertes der Kultur oder des Mycelvolumens sowie durch Dünnschichtchromatographie oder Ausprüfen der biologischen Aktivität überwacht werden. Die Verbindung der allgemeinen Formel I ist sowohl im Mycel als auch im Kulturfiltrat enthalten.

Die Isolierung der genannten Verbindung aus dem Kulturmedium erfolgt nach bekannten Methoden unter Berücksichtigung der chemischen, physikalischen und biologischen Eigenschaften der Produkte. Zum Testen der Antibiotika-Konzentration im Kulturmedium oder in den einzelnen Isolierungsstufen kann die Dünnschichtchromatographie, beispielsweise auf Kieselgel mit Chloroform/Methanol als Laufmittel verwendet werden, wobei die Menge der gebildeten antibakteriellen Substanz zweckmäßig mit einer Eichlösung verglichen wird.

Zur Isolierung der Verbindung werden Kulturbrühe und Mycel zuerst mit organischen Lösungsmitteln, wie z. B. Chloroform, Essigester usw. extrahiert, um die unpolaren Verunreinigungen zu entfernen. Anschließend wird mit einem polaren Lösungsmittel, beispielsweise niederen Alkanolen oder Gemischen aus Chloroform und/oder Essigester mit einem niederen Alkanol, extrahiert.

Die Reinisolierung erfolgt vorzugsweise an geeigneten Medien, wie z. B. Kieselgel, Aluminiumoxid oder Ionenaustauschern, durch anschließende Elution mit organischen, polaren Lösungsmitteln oder Lösungsmittelgemischen, wie z. B. Essigsäurealkylester, Gemische aus Essigsäurealkylester und einem niederen Alkanol, gegebenenfalls mit Wasser, bzw. einem für Ionenaustauscher geeigneten Salzgradienten, wie beispielsweise Kochsalz oder Tris(hydroxymethyl)aminomethan-HCl (Tris-Puffer), und Sammeln der antibiotisch wirksamen Fraktionen.

Die Verbindungen sind in festem Zustand und in Lösungen im pH-Bereich 2 bis 8, insbesondere 3 bis 7, stabil und lassen sich damit in übliche galenische Zubereitungen einarbeiten.

In den folgenden Beispielen wird die Erfindung in weiteren Details beschrieben. Prozentangaben beziehen sich auf das Gewicht, wenn nicht anders angegeben.

**Beispiele:**

1. a) Herstellung einer Sporensuspension des Produzentenstammes:

100 ml Nährlösung (2 g Hefeextrakt, 20 g Malzextrakt, 10 g Glucose, 0,5 g $(NH_4)_2HPO_4$, 1 l Leitungswasser, pH-Wert vor dem Sterilisieren 7,3) in einem 500 ml Erlenmeyerkolben werden mit dem Stamm DSM 4209 oder DSM 4210 beimpft und 72 Stunden bei 25 °C und 120 UpM auf der rotierenden Schüttelmaschine inkubiert. Anschließend werden 20 ml Kulturflüssigkeit in einem 500 ml Erlenmeyerkolben mit dem Nährboden der obengenannten Zusammensetzung, dem 20 g Agar/l zur Verfestigungen zugegeben wurde, gleichmäßig verteilt und dekantiert.

Die Kulturen werden 10 bis 14 Tage bei 25 °C inkubiert. Die nach dieser Zeit entstandenen Sporen eines Kolbens werden mit 500 ml entionisiertem Wasser, das einen Tropfen eines handelsüblichen nichtionischen Tensids (Triton X 100, Fa. Serva) enthält, abgeschwemmt, sofort weiterverwendet oder bei

-22 °C aufbewahrt.

b) Herstellung einer Kultur bzw. Vorkultur des Produzentenstammes im Erlenmeyerkolben.

Ein 500 ml Erlenmeyerkolben mit 100 ml der unter a) beschriebenen Nährlösung wird mit einer auf einem Schrägröhrchen gezogenen Kultur oder mit 0,2 ml Sporensuspension angeimpft und auf einer Schüttelmaschine bei 120 UpM und 25 °C inkubiert. Die maximale Produktion der Verbindung der Formel I ist nach ca. 120 Stunden erreicht. Zum Animpfen von 10 und 100 l Fermentern genügt eine 48 Stunden alte Submerskultur (5 %) aus der gleichen Nährlösung.

2. Herstellung der Verbindung der allgemeinen Formel I

Ein 10 l Fermenter wird unter folgenden Bedingungen betrieben:

| | |
|---|---|
| Nährmedium: | 20 g/l Malzextrakt<br>2 g/l Hefeextrakt<br>10 g/l Glucose<br>0,5 g/l $(NH_4)_2PO_4$<br>pH 7,2 |
| Inkubationszeit:<br>Inkubationstemperatur:<br>Rührergeschwindigkeit:<br>Belüftung: | 150 Stunden<br>25 °C<br>250 UpM<br>4 l Luft/min. |

Durch wiederholte Zugabe weniger Tropfen ethanolischer Polyollösung kann die Schaumentwicklung unterdrückt werden. Das Produktionsmaximum wird nach ca. 150 Stunden erreicht. Die Ausbeuten liegen bei ca. 100 mg/l.

3. Isolierung der Verbindung der allgemeinen Formel I

Nach der Fermentation von DSM 4209 bzw. DSM 4210 wird die Kulturbrühe unter Zusatz von 2 % Celite als Filterhilfsmittel filtriert. Es wird nach den folgenden Schemata verfahren:

**Aufarbeitung / Isolierung**

Schema 1: Kulturüberstand

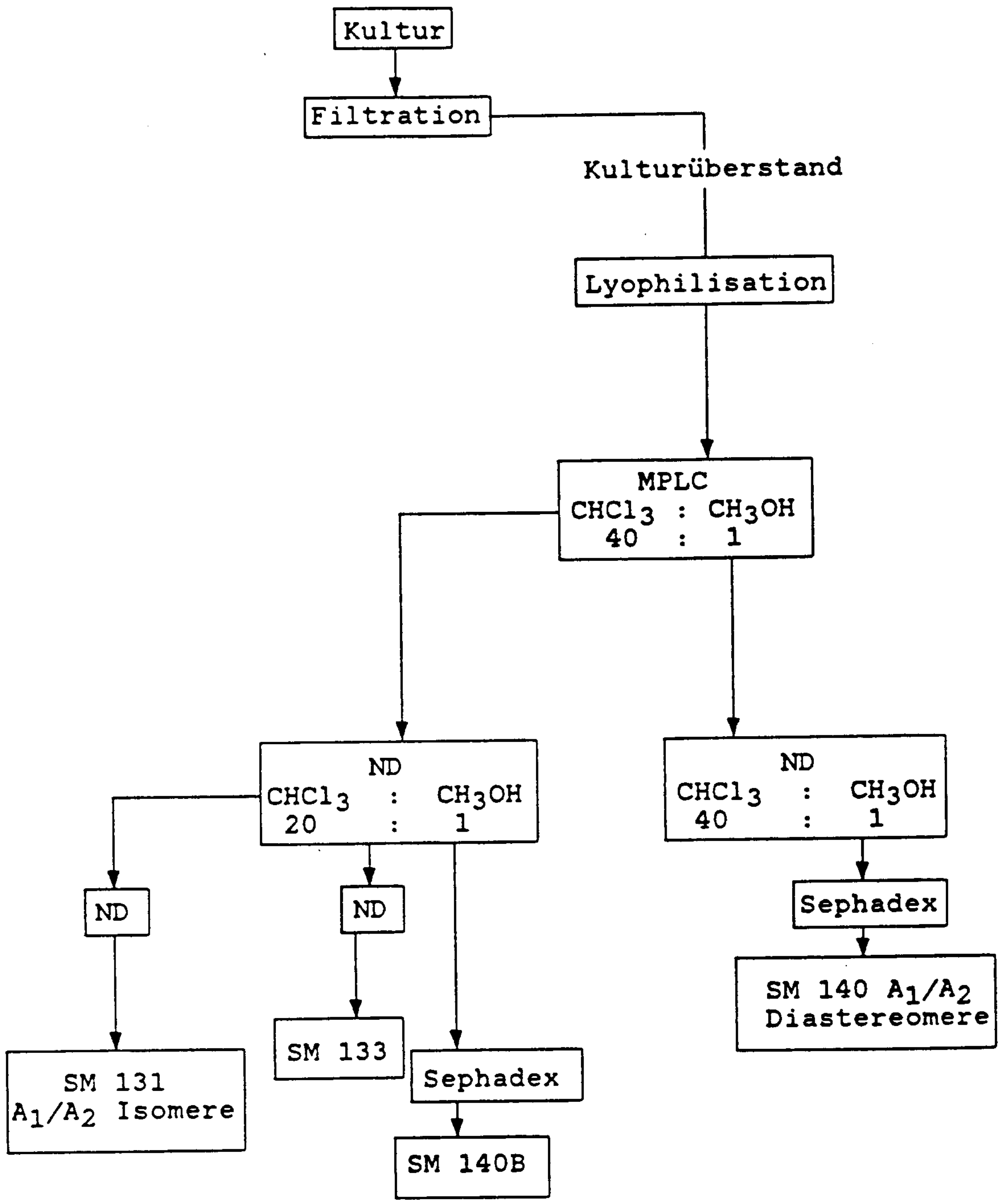

**Aufarbeitung / Isolierung**

Schema 2: Mycel

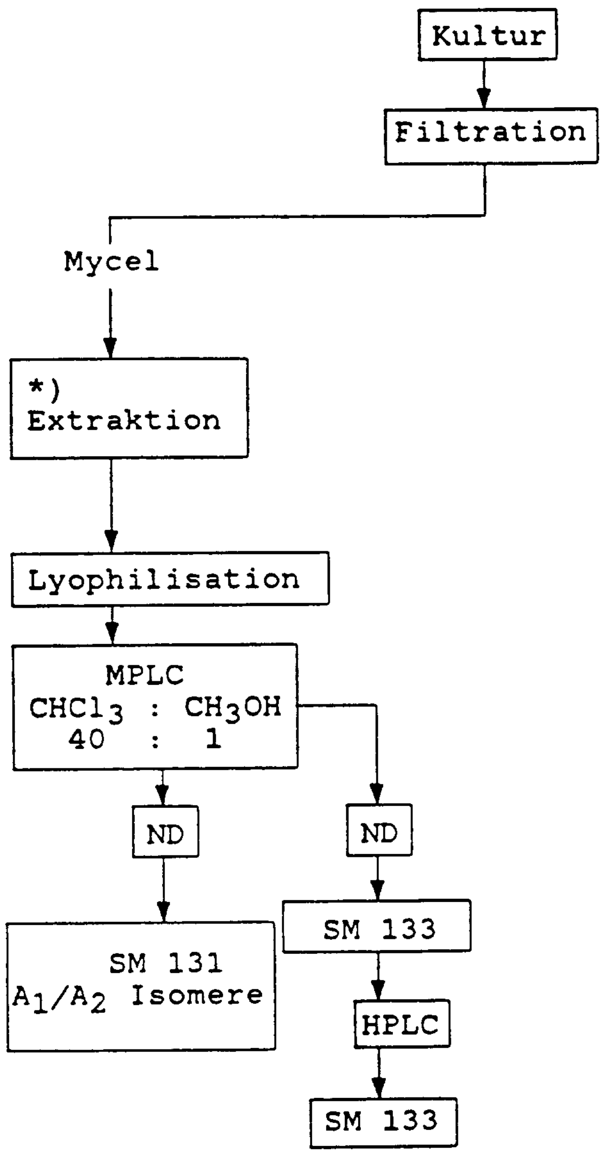


Abkürzungen:

MPLC: Mitteldruck-Flüssigchromatographie

ND: Normaldruck-Säulenchromatographie

*) eventuell nach Aufschluß mit Homogenisatoren

**1. Charakterisierung der Verbindungen SM 131**

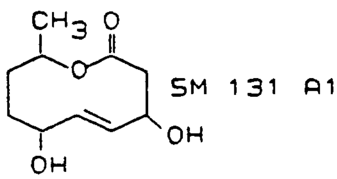
SM 131 A1

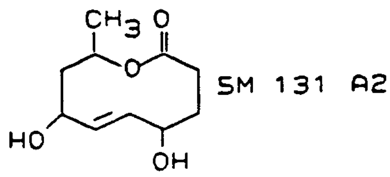
SM 131 A2

:

Die Verbindungen SM 131 A1 und A2 liegen als Isomerenmischung (ca. 1:1) vor und wurden nicht getrennt.

Dünnschichtchromatographie:

Kieselgel 60, $F_{254}$: Chloroform/Methanol (9:1, v:v): Rf 0,40 n-Butanol/Essigsäure/Wasser (Oberphase) (4:1:5, v:v:v): Rf 0,60

EI-MS (70 eV) vom Bis-trimethylsilylether: m/e = 344 ($M^+$, 1 %); 274 (M + H-$Me_3Si$, 1 %); 201 (M + H-$2Me_3Si$) entsprechend $C_{10}H_{16}O_4$ (200,24) für SM 131 A1/A2

Schmp. 100 °C

IR (KBr): 3400, 2980, 2940, 2870, 2860, 1720, 1700 sh $cm^{-1}$

UV (MeOH): Endabsorption

$^1$H-NMR (200 MHz, $CD_3OD$): SM 131 A1 + A2

$\delta$ = 1.15 (d, 3H, J = 6,3 Hz, 9-$CH_3$); 1,20 (d, 3H, J = 6,7 Hz, 9-$CH_3$); 1,43 (dd, 1H, J = 16/7,5 Hz); 1,6-1,8 (m, 5H); 1,8-2,0 (m, 2H); 2,30 (dd, 1H, J = 13,4/5,6 Hz); 2,50 (m, 2H); 2,92 (dd, 1H, J = 13,4/7,7 Hz); 3,94 (m, 1H); 4,38 (m, 1H); 4,56 (dddd, 1H, J = 7,7/6,8/5,6/1 Hz); 4,66-4,78 (m, 2H); 4,99 (dq, 1H, J = 6,7/3,5 Hz); 5,40 + 5,55 (AB-System, je 1 dd, 2H J = 16/8,2 und 16/6,8 Hz, Olefin-H); 5.77 + 5.90 (AB-System, je 1 ddd, 2H, J = 16/2,3/1 und 16/2,8/1,4 Hz, Olefin-H) ppm

$^{13}$C-NMR (50.3 MHz, $CD_3OD$):

$\delta$ = 18,9 g (9-$CH_3$; 22,1 g (9-$CH_3$); 28,6 t; 29,2 t; 32,6 t; 36,2 t; 45,3 t (C-2); 45,8 t (C-2); 68,7 d; 69,0 d; 70,3 d; 72,3 d; 74,1 d; 130,6 d; 131,9 d; 138,2 d; 172,0 s (C-1); 172,4 s (C-1) ppm.

| | | | |
|---|---|---|---|
| Elementaranalyse: | Berechnet:<br>Gefunden: | C 60,00<br>C 60,40 | H 8,00<br>H 8,09 |

**2. Charakterisierung der Verbindung SM 133**

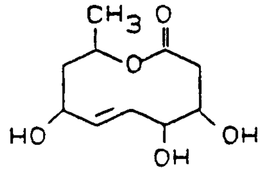

Dünnschichtchromatographie:

Kieselgel 60, $F_{254}$: Chloroform/Methanol (9:1, v:v): Rf 0,30 n-Butanol/Essigsäure/Wasser, obere Phase (4:1:5): Rf 0,60

EI-MS (70 eV): kein Molekülion, m/e = 110 ($C_7H_{10}O$, 40 %); 86 ($C_4H_6O_2$, 100 %)

IR (Film): 3400, 2970, 2930, 2905, 1715 sh, 1700, 1640 $cm^{-1}$

UV (MeOH): Endabsorption

$^1$H-NMR (200 MHz, $CDCl_3$):

$\delta$ = 1,25 (d, 3H, J = 6,5 Hz, 9-$CH_3$); 1,67 (ddd, 1H, J = 14/10,8/10,8 Hz, 8-$H_a$); 1,82 (ddd, 1H, J = 14/3,8/2 Hz, 8-$H_b$); 2,29 (dd, 1H, J = 14,2/7,2 Hz, 2-$H_a$); 2,53 (dd, 1H, J = 14,2/2,8 Hz, 2-$H_b$); 3,0-3,7 (s, br, 3H, 3 OH, tauschen aus); 3,95 (ddd, 1H, J = 7,2/5/2,8 Hz, 3-H); 4,07 (dddd, 1H, J = 10,8/7,2/3,8/2,8 Hz, 7-H); 4,13 (ddd, 1H, J = 5/2/1 Hz, 4-H); 5,20 (ddg, 1H J = 10,8/6,5/2 Hz, 9-H); 5,7-5,94 (m, 2H, AB-System,

5-H, 6-H) ppm.
$^{13}$C-NMR (50.3 MHz, $CD_3OD$):
$\delta$ = 21,7 q (9-$CH_3$); 35,2 t (C-8); 44,1 t (C-2); 69,3 d; 73,0 d; 73,5 d; 75,3 d; 129,4 d; 135,8 d; 174,7 s (C-1) ppm.
$[\alpha]_D^{20}$ (c = 1, Methanol): -62°

| | | | |
|---|---|---|---|
| Elementaranalyse: | Berechnet:<br>Gefunden: | C 55,55;<br>C 55,73; | H 7,41 (für $C_{10}H_{16}O_5$,MG 216,24)<br>H 7,48 |

**3. Charakterisierung der Verbindungen SM 140 $A_1/A_2$ und SM 140 B**

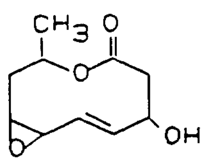
SM 140 $A_1/A_2$

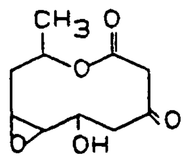
SM 140 B

Mischung von Diastereomeren
im Verhältnis $A_1/A_2 \approx 2:1$
Dünnschichtchromatographie:
Kieselgel 60, $F_{254}$, Chloroform/Methanol (9:1, v:v): Rf 0.60 n-Butanol/Essigsäure/Wasser (obere Phase) (4:1:5): Rf 0.80
Verbindung SM 140 $A_1/A_2$
Positiv-fast atom bombardment-MS: m/e = 199 ($M+H^+$, 84 %) entsprechend $C_{10}H_{14}O_4$ (198,22)
IR (KBr): 3460, 3390, 3005, 2965, 2940, 2915, 2900, 2875, 1715, 1680 $cm^{-1}$
UV (MeOH): Endabsorption
$^1$H-NMR (200 MHz, $CDCl_3$):
Signale A1:
$\delta$ = 1,45 (d, 3H, J = 7 Hz, 9-$CH_3$); 1,95 (ddd, 1H, J = 15/11/10 Hz, 8-$H_a$); 2,12 (ddd, 1H, J = 15/5/1,5 Hz, 8-$H_b$); 2,33 (dd, 1H, J = 11,9/9,5 Hz, 2-$H_a$); 2,83 (dd, 1H, J = 11,9/6,6 Hz, 2-$H_b$); 3,05 (ddd, 1H, J = 10/5/4,2 Hz, 7-H); 3,57 (dd, 1H, J = 8/4,2 Hz, 6-H); 4,65 (ddq, 1H, J = 11/7/1 Hz, 9-H); 4,65 (ddd, 1H, J = 9,5/8,6/6,6 Hz, 3-H); 5,42 (dd, 1H, J = 16,8/8 Hz, 5-H); 5,84 (dd, 1H, J = 16,8/8,6 Hz, 4-H) ppm.
Signale A2:
$\delta$ = 1,39 (d, 3H, J = 6,8 Hz, 9-$CH_3$); 1,83 (ddd, 1H, J = 16/6,5/4,8 Hz, 8-$H_a$); 2,22 (m, 1H, 8-$H_b$); 2,43 (dd, 1H, J = 12/3 Hz, 2-$H_a$); 2,63 (dd, 1H, J = 12/4,2 Hz, 2-$H_b$); 3,19 (ddd, 1H, J = 6,5/5/4 Hz, 7-H); 3,50 (m, 1H, 6-H); 4,8 (m, 2H, 3-H, 9-H); 5,77 (ddd, 1H, J = 15,5/2/1 Hz, 5-H); 6,07 (ddd, 1H, J = 15,5/1,8/1,5 Hz, 4-H) ppm.
$^{13}$C-NMR (50.3 MHz, $CDCl_3$):
Signale $A_1$:
$\delta$ = 21,5 q (9-$CH_3$); 34,6 t (C-8); 45,7 t (C-2); 55,0 d; 55,2 d; 71,6 d; 71,7 d; 129,7 d; 133,9 d; 170,7 s (C-1) ppm.
Signale $A_2$:
$\delta$ = 19.0 q (9-$CH_3$); 29,0 t (C-8); 44,4 t (C-2); 53,5 d; 54,1 d; 68,2 d; 69,3 d; 120,1 d; 136,7 d; 171,1 s (C-1) ppm.
Verbindung SM 140 B
DCJ-MS: m/e = 249 ($M + NH_3 + NH_4^+$, 100 %) entsprechend $C_{10}H_{14}O_5$ (214.22)
IR (KBr): 3410, 3350, 3000, 2980, 2940, 1740, 1700 $cm^{-1}$
UV (Methanol): Endabsorption
$^1$H-NMR (200 MHz, $CDCl_3$):

δ = 1,34 (d, 3H, J = 6,3 Hz, 9-$CH_3$); 1,53 (ddd, 1H, J = 14,5/10,4/11,4 Hz, 8-$H_a$); 2,36 (ddd, 1H, J = 14,5/4,3/1,2 Hz, 8-$H_b$); 2,58 (d, 1H, J = 2,4 Hz, tauscht aus, 5-OH); 2,79 (dd, 1H, J = 13,2/3,7 Hz, 4-$H_a$); 2,87 (dd, 1H, J = 13,2/6 Hz, 4-$H_b$); 3,00 (dd, 1H, J = 9/4 Hz, 6-H); 3,19 (ddd, 1H, J = 10,4/4,3/4 Hz, 7-H); 3,48 (dd, 2H, AB-System, J = 14,5 Hz, 2-$H_2$); 3,83 (dddd, 1H, J = 9/6/3,7/2,4 Hz, 5-H);5,15 (ddq, 1H, J = 11,4/6,3/1,2 Hz, 9-H) ppm.

$^{13}$C-NMR (50,3 MHz, $CDCl_3$):

δ = 20,6 g (9-$CH_3$); 36,5 t (C-8); 48,4 t (C-4); 51,9 t (C-2); 56,0 d; 60,5 d; 67,7 d; 69,1 d; 165,4 s (C-1); 200,8 s (C-3) ppm.

## Patentansprüche

**1.** Die Verbindung der allgemeinen Formel I,

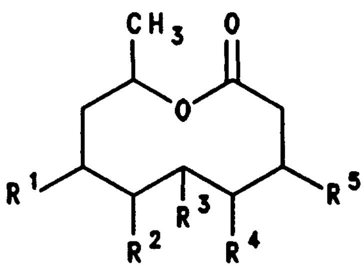

I

in der
unabhängig voneinander

| | |
|---|---|
| $R^1$ | Wasserstoff oder Hydroxyl, |
| $R^2$ | Hydroxyl oder |
| $R^1$ und $R^2$ | zusammen mit den tragenden C-Atomen einen Oxiranring darstellen, |
| $R^3$ | Hydroxyl oder |
| $R^2$ und $R^3$ | gemeinsam eine Doppelbindung darstellen, |
| $R^4$ | Wasserstoff oder Hydroxyl oder |
| $R^3$ und $R^4$ | gemeinsam eine Doppelbindung darstellen und |
| $R^5$ | Wasserstoff, Hydroxyl oder eine Oxogruppe bedeuten. |

**2.** Das Verfahren zur Herstellung der Verbindung der allgemeinen Formel I nach Anspruch 1, dadurch gekennzeichnet, daß Penicillium sp. DSM 4209 und/oder DSM 4210 in einem Nährmedium kultiviert wird, bis sich die Verbindung der allgemeinen Formel I in der Kultur anhäuft.

**3.** Verfahren nach Anspruch 2, dadurch gekennzeichnet, daß das Nährmedium 0,2 bis 5 % Malzextrakt, 0,02 bis 0,5 % Hefeextrakt, 0,1 bis 5 % Glucose sowie 0,005 bis 0,2 % Ammoniumsalz enthält, jeweils bezogen auf das Gewicht der gesamten Nährlösung.

**4.** Verfahren nach Anspruch 3, dadurch gekennzeichnet, daß das Nährmedium 1 bis 4 % Malzextrakt, 0,1 bis 0,4 % Hefeextrakt, 0,5 bis 2 % Glucose und 0,01 bis 0,1 % Ammoniumsalz enthält.

**5.** Verfahren nach einem oder mehreren der Ansprüche 2 bis 4, dadurch gekennzeichnet, daß die Kultivierung bei 18 bis 35 °C erfolgt.

**6.** Verfahren nach einem oder mehreren der Ansprüche 2 bis 5, dadurch gekennzeichnet, daß die Kultivierung in dem pH-Bereich zwischen 6 und 8 erfolgt.

**7.** Verwendung der Verbindung der allgemeinen Formel I nach Anspruch 1 zur Herstellung eines Arzneimittels für die therapeutische Anwendung.

**8.** Verwendung der Verbindung der allgemeinen Formel I nach Anspruch 1 zur Herstellung eines antibakteriell wirkenden Arzneimittels.

9. Penicillium sp. DSM 4209 und DSM 4210, sowie deren Varianten und Mutanten soweit sie die Verbindung der allgemeinen Formel I nach Anspruch 1 herstellen.

## Claims

1. A compound of the formula I

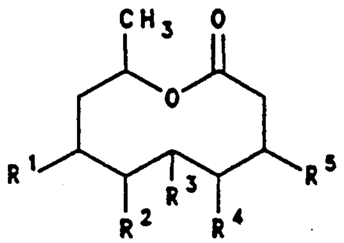

I

in which, independently of one another,

| | |
|---|---|
| $R^1$ | denotes hydrogen or hydroxyl, |
| $R^2$ | denotes hydroxyl, or |
| $R^1$ and $R^2$ | represent, together with the carrying carbon atoms, an oxirane ring, |
| $R^3$ | denotes hydroxyl, or |
| $R^2$ and $R^3$ | together represent a double bond, |
| $R^4$ | denotes hydrogen or hydroxyl, or |
| $R^3$ and $R^4$ | together represent 2 double bond, and |
| $R^5$ | denotes hydrogen, hydroxyl or an oxo group. |

2. The process for the preparation of a compound of the formula I as claimed in claim 1, which comprises culturing Penicillium sp. DSM 4209 and/or DSM 4210 in a nutrient medium until a compound of the formula I accumulates in the culture.

3. The process as claimed in claim 2, wherein the nutrient medium contains 0.2 to 5% malt extract, 0.02 to 0.5% yeast extract, 0.1 to 5% glucose and 0.005 to 0.2% ammonium salt, in each case based on the weight of the complete nutrient solution.

4. The process as claimed in claim 3, wherein the nutrient medium contains 1 to 4% malt extract, 0.1 to 0.4% yeast extract, 0.5 to 2% glucose and 0.01 to 0.1% ammonium salt.

5. The process as claimed in one or more of claims 2 to 4, wherein the culturing is carried out at 18 to 35 °C.

6. The process as claimed in one or more of claims 2 to 5, wherein the culturing is carried out in the pH range between 6 and 8.

7. The use of a compound of the formula I as claimed in claim 1 for producing a pharmaceutical for therapeutic administration.

8. The use of a compound of the formula I as claimed in claim 1 for producing an antibacterial pharmaceutical.

9. Penicillium sp. DSM 4209 and DSM 4210, as well as the variants and mutants thereof as long as they produce a compound of the formula I as claimed in claim 1.

## Revendications

**1.** Composé de formule générale I

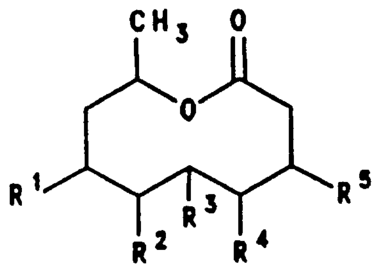

I

dans laquelle, indépendamment les uns des autres,

| | |
|---|---|
| $R^1$ | représente un atome d'hydrogène ou le groupe hydroxy, |
| $R^2$ | représente le groupe hydroxy ou |
| $R^1$ et $R^2$ | représentent ensemble, avec les atomes de carbone qui les portent, un cycle oxyrane, |
| $R^3$ | représente le groupe hydroxy ou |
| $R^2$ et $R^3$ | représentent ensemble une double liaison, |
| $R^4$ | représente un atome d'hydrogène ou le groupe hydroxy ou |
| $R^3$ et $R^4$ | représentent ensemble une double liaison et |
| $R^5$ | représente un atome d'hydrogène ou le groupe hydroxy ou oxo. |

**2.** Procédé pour la production du composé de formule générale I selon la revendication 1, caractérisé en ce que l'on cultive *Penicillium sp.* DSM 4209 et/ou DSM 4210 dans un milieu de culture, jusqu'à ce que le composé de formule générale I s'accumule dans la culture.

**3.** Procédé selon la revendication 2, caractérisé en ce que le milieu nutritif contient 0,2 à 5 % d'extrait de malt, 0,02 à 0,5 % d'extrait de levure, 0,1 à 5 % de glucose ainsi que 0,005 à 0,2 % de sel d'ammonium, dans chaque cas par rapport au poids de la solution nutritive totale.

**4.** Procédé selon la revendication 3, caractérisé en ce que le milieu nutritif contient 1 à 4 % d'extrait de malt, 0,1 à 0,4 % d'extrait de levure, 0,5 à 2 % de glucose et 0,01 à 0,1 % de sel d'ammonium.

**5.** Procédé selon une ou plusieurs des revendications 2 à 4, caractérisé en ce que la culture est effectuée à 18-35 °C.

**6.** Procédé selon une ou plusieurs des revendications 2 à 5, caractérisé en ce que la culture est effectuée dans l'intervalle de pH compris entre 6 et 8.

**7.** Utilisation du composé de formule générale I selon la revendication 1, pour la fabrication d'un médicament pour utilisation thérapeutique.

**8.** Utilisation du composé de formule générale I selon la revendication 1, pour la fabrication d'un médicament à action antibactérienne.

**9.** *Penicillium sp.* DSM 4209 et DSM 4210, ainsi que leurs variants et mutants, dans la mesure où ils produisent le composé de formule générale I selon la revendication 1.